# EUROPÄISCHE PATENTSCHRIFT

(11) **EP 0 931 522 B1**
(45) Veröffentlichungstag und Bekanntmachung des Hinweises auf die Patenterteilung: **17.03.2004**
(21) Anmeldenummer: 98810021.0
(22) Anmeldetag: 16.01.1998
(51) Int. Cl.: A61F 2/46, A61F 2/40

(54) **Baukasten für Schaftprothesen**
Assembling set for shaft prostheses
Ensemble d'assemblage pour prothèses à tige

(43) Veröffentlichungstag der Anmeldung: 28.07.1999
(73) Patentinhaber: Centerpulse Orthopedics Ltd., 6340 Baar (CH)
(72) Erfinder: Farey, Samuel, 25460 Etupes (FR)
(74) Vertreter: Manitz, Finsterwald & Partner Gbr

(56) Entgegenhaltungen:
- EP-A- 0 163 121
- EP-A- 0 373 078
- WO-A-94/15551
- WO-A-97/25943
- FR-A- 2 727 857
- US-A- 4 676 798
- US-A- 5 122 145
- US-A- 5 129 907
- US-A- 5 312 216
- US-A- 5 645 607

## Beschreibung

Die Erfindung betrifft einen Baukasten mit einer Montagevorrichtung zum Montieren von Schaftprothesen gemäß Anspruch 1 sowie eine Montageeinrichtung für einen solchen Baukasten gemäß Anspruch 6.

Die FR-A-2 727 857 zeigt eine Schaftprothese für ein Schultergelenk, die aus einem Stiel und aus einem Prothesenkopf besteht, die über ein festsetzbares Kugelgelenk miteinander verbunden sind. Der Prothesenkopf besteht aus einem flachen Kugelausschnitt, dessen ebene Unterseite auf einer ebenen Resektionsfläche eines Humerus aufliegen soll, um die Resektionsfläche vollständig abzuschliessen. Ein vom Stiel in einer schrägen Richtung zur Stielachse wegstehender Kugelkörper ist durch Schlitze in Lappen aufgeteilt, die durch einen Dorn, der in der schrägen Richtung durch den Stiel hindurch in den Kugelkörper hineingetrieben wird, aufspreizbar sind. Im Prothesenkopf ist eine kugelförmige Lagerschale von der Unterseite eingearbeitet, die den Kugelkörper beim Aufspreizen umfängt und in einer wählbaren Neigung zur Stielachse blockiert. Ein Nachteil dieser Anordnung ist, dass diese Einstellung der Neigung im voraus erfolgen muss und dem Operateur ein grosses räumliches Vorstellungsvermögen abverlangt. Da die Abmessungen für das festsetzbare Kugelgelenk aus Platzgründen klein gehalten werden müssen, ist beim Festsetzen der Lappen zwangsweise eine plastische Verformung an deren Füssen notwendig und bei einem Spreizdorn zur Sicherheit Selbsthemmung notwendig. Das heisst, der Operateur hat bei allen anderen Vorteilen dieser Konstruktion, eigentlich nur einen Versuch, um die Kupplung definitiv festzusetzen.

US 5,645,607 beschreibt einen Probierschaft für eine Hüftprothese, bei dem ein Halsabschnitt relativ zu einem fest mit einem Schaftabschnitt verbundenen Basisabschnitt verstellbar ist.

Aufgabe der Erfindung ist es, eine genaue Voreinstellung einer einen relativ zu einem Stiel verstellbaren Kopf umfassenden Schaftprothese zu erreichen.

Die Lösung dieser Aufgabe erfolgt zum einen durch den Baukasten gemäß Anspruch 1 und zum anderen durch die Montagevorrichtung gemäß Anspruch 6.

Ein Vorteil der Anordnung gemäß Anspruch 1 besteht darin, dass durch das Baukastenprinzip weniger Teile bei Prothesen und Probierprothesen notwendig sind, um ein grosses Spektrum abzudecken. Ein weiterer Vorteil besteht darin, dass die Probierprothese beim Einsetzen mit ihrem Kopf beweglich zum Stiel gelagert ist, um den Kopf in die günstigste Lage bezüglich Resektionsfläche und Artikulation zu bringen. Die Stellung vom Kopf ist im Knochen eingesetzten Zustand festsetzbar und seine Funktion überprüfbar. Bei eingesetztem Stiel der Probierprothese kann der Kopf ausgewechselt, der neue Kopf ausgerichtet und festgesetzt werden und seine Funktion überprüft werden. Der Operateur hat die Sicherheit, dass die später eingesetzte Schaftprothese ihre Funktion gleich gut erfüllt.

Weitere vorteilhafte Ausführungen für den Baukasten ergeben sich aus den abhängigen Ansprüchen 2 bis 5. So ist die Anwendung für Schultergelenke günstig, die eine vergleichsweise geringere Belastung aufweisen und daher als Kupplung vom Stiel zum Kopf festsetzbare Kugelgelenke zulassen, deren Lagerschale im Prothesenkopf angeordnet ist und trotz verschiedener Aussendimensionen des Kopfes die gleiche Lage zur Unterseite aufweist. Durch einen radialen Versatz vom Zentrum der Lagerschale zur Mittelachse des Kopfes kann dieser durch Drehung in seinem Abstand zur Stielachse verändert werden und so ein grösseres Spektrum von zur Stielachse unterschiedlich versetzten Resektionsflächen abdecken.

Vorteilhafte Weiterbildungen der Montagevorrichtung sind in den abhängigen Ansprüchen 7 bis 11 gezeigt. Eine derartige Vorrichtung kann nicht nur zum Einstellen von Schaftprothesen gemäss von in ihrer Funktion überprüften Probierprothesen verwendet werden. Sie gestattet es auch bei Frakturen des Knochens, die Probierprothesen verunmöglichen, die Schaftprothesen in einer vorgesehenen Winkelstellung, beispielsweise in einem Zwischenwinkel zwischen einer zur Mittelachse des Kopfes parallelen Kupplungsachse und Stielachse von 130° und einer Retrotorsion links oder rechts von 18° einzustellen.

Statt einer schwimmenden Scheibe, die in einer Kugelführung gelagert ist, könnte auch ein festsetzbares Kardangelenk in einer derartigen Montagevorrichtung verwendet werden. Grundsätzlich muss die zweite Aufnahmevorrichtung so beschaffen sein, dass sie den durch die Kupplung möglichen Verstellbewegungen folgen kann.

Im folgenden wird die Erfindung anhand von Ausführungsbeispielen beschrieben. Es zeigen:
- Fig. 1: Schematisch einen Humerus mit einem Gelenkkopf;
- Fig. 2: schematisch eine Schaftprothese mit einem im Humerus einsetzbaren Stiel und einem daran festsetzbaren Kopf;
- Fig. 3: schematisch eine Schaftprothese von Figur 2, die in einen Humerus nach Figur 1 eingesetzt ist;
- Fig. 4: schematisch eine Seitenansicht von verschieden grossen Prothesenköpfen für eine Humerusprothese nach Figur 2;
- Fig. 5: schematisch in zwei Ansichten verschieden grosse Prothesenstiele, die mit Prothesenköpfen der Figur 4 kombinierbar sind;
- Fig. 6: schematisch eine in einem Humerus eingesetzte Probierprothese;
- Fig. 7: schematisch ein Ausziehwerkzeug für eine Probierprothese nach Figur 6 mit einem daran ansetzbaren Gleithammer;
- Fig. 8: schematisch eine Explosionszeichnung von einer Montagevorrichtung für Humerusprothesen;
- Fig. 9: schematisch die Montagevorrichtung von Figur 8 mit einer eingesetzten Probierprothese;
- Fig. 10: schematisch die Montagevorrichtung von Figur 8 mit einer eingesetzten Schaftprothese und mit einem Drehmomentschlüssel beim Festsetzen des Prothesenkopfes;
- Fig. 11: schematisch eine im Humerus eingesetzte Probierprothese beim Fixieren des Prothesenkopfes in einer bevorzugten Stellung zum Prothesenstiel;
- Fig. 12: schematisch eine lose in eine Montagevorrichtung von Figur 8 eingelegte Probierprothese vor dem Einspannen des Prothesenstiels;
- Fig. 13: schematisch ein Ausrichten der Neigung einer schwimmenden Scheibe zur Unterseite vom Kopf einer am Stiel eingespannten Probierprothese; und
- Fig. 14: schematisch ein Nachfahren der Anzeige für die Lage vom Versatz zwischen Mittelachse und Lagerschalenzentrum am Kopf einer Probierprothese der Figur 13.

In den Figuren ist ein Baukasten zum Montieren von Schaftprothesen gezeigt, welche eine in einem vorbestimmten Winkelbereich festsetzbare Kupplung zwischen Prothesenstiel 3 und Prothesenkopf 4 aufweisen, die durch eine Vorrichtung im Prothesenstiel 3 festsetzbar ist. Durch einen Baukasten mit kombinierbaren verschieden grossen Stielen und Prothesenköpfen für äusserlich und in der Lage der Drehpunkte baugleiche Prothesen 3, 4 und Probierprothesen entsteht eine Vielfalt von Probierprothesen und Schaftprothesen. Die Probierprothesen besitzen eine Vorrichtung im Prothesenkopf, die das Festsetzen der Kopfes bei in einen Knochen eingesetzten Stiel in einer optimalen Stellung mit kontrollierbarer Funktion erlauben. Diese Stellung zwischen Kopf und Stiel wird beim Ziehen der Probierprothese beibehalten und auf eine Montagevorrichtung übertragen 1, in der eine aus analogen Teilen aufgebaute Schaftprothese 3, 4 in die gleiche Stellung gebracht und fixiert wird.

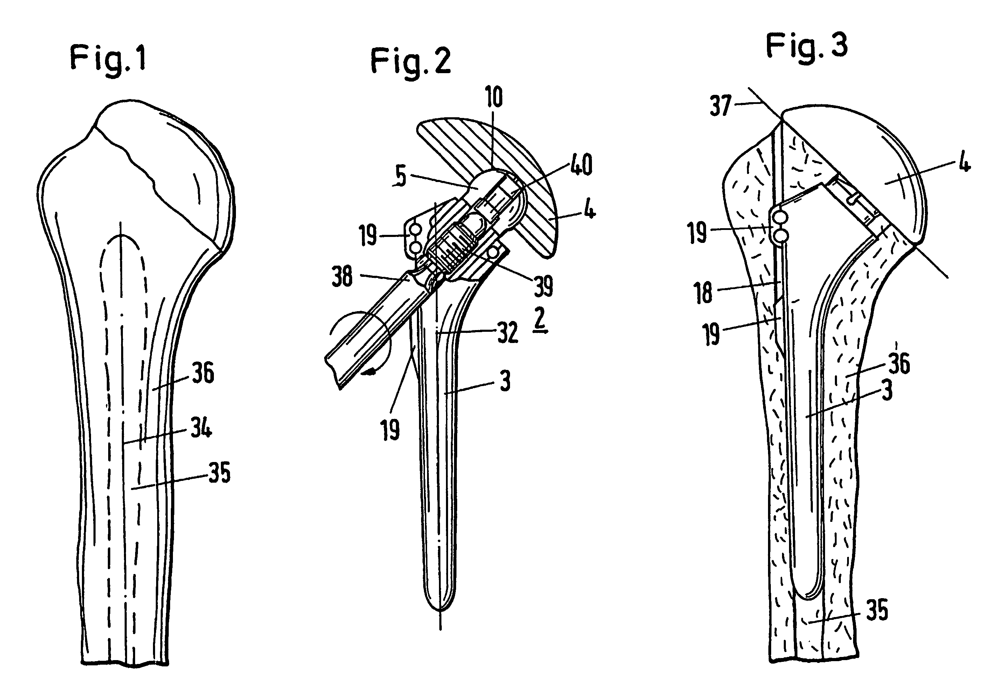

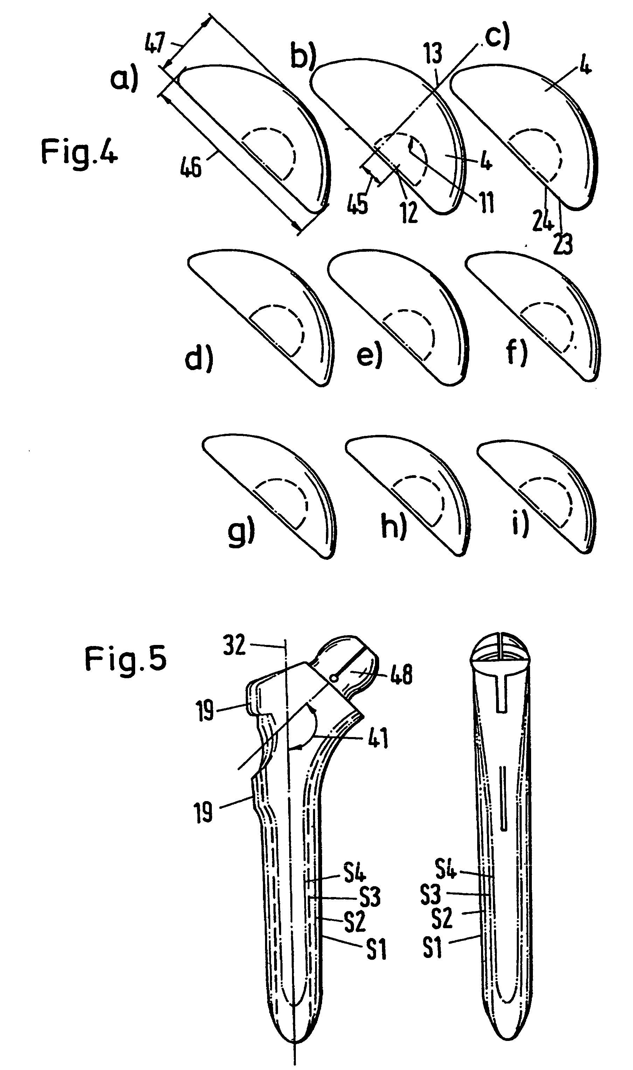

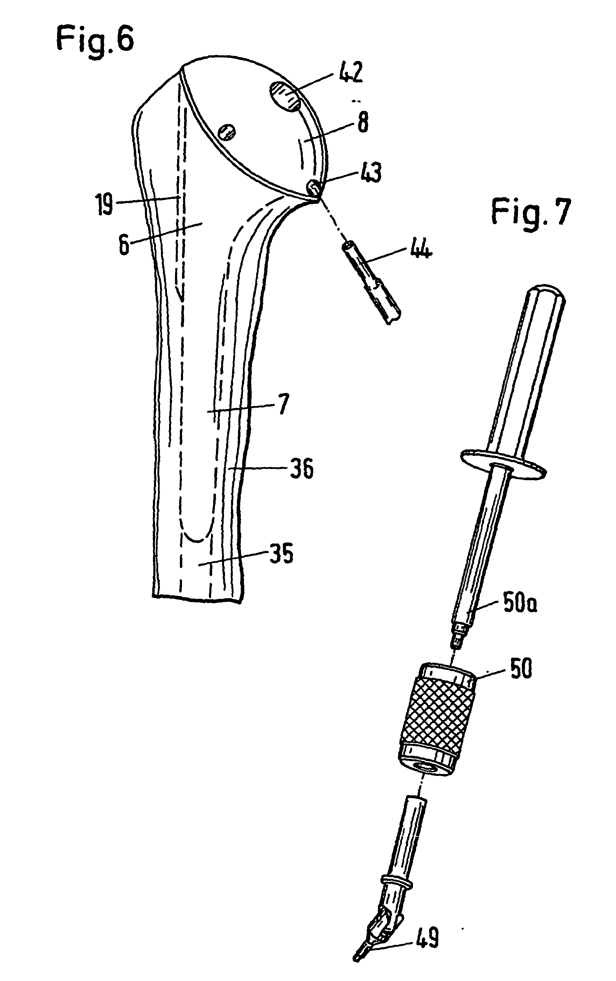

In den Figuren 1 bis 5 sind Schaftprothesen 2 für einen Humerus 36 gezeigt, die mit einem Stiel 3 in dem erweiterten Markkanal 35 des Humerus verankert sind. Der erweiterte Markkanal ist mit einer Nut 18 versehen, die eine Finne 19 vom Stiel 3 führt und dem Stiel eine eindeutige Lage verleiht. Stielachse 32 und Achse 34 vom Markkanal fallen ungefähr zusammen. Der Prothesenschaft besteht aus einer warmgeschmiedeten Legierung beispielsweise nach ISO 5832-9 und besitzt vier Grössen S₁ bis S₄ (Figur 5). Der Stiel 3 hat Trompetenform, um ein gezieltes Verblocken beim Einsetzen zu erreichen. Rotationsstabilität wird durch die laterale Finne 19 im proximalen Bereich erreicht. Ein Kugelkopf bildet die Kupplung 5 zum Prothesenkopf 4. Er bildet mit der Schaftachse 32 einen Winkel 41 von 130°. Der Kugelkopf ist durch den Stiel hindurch mit einer Bohrung versehen und durch Längsschlitze in vier abbiegbare Lappen 48 unterteilt. Ein Aufspreizen der Lappen 48 erfolgt durch einen Expansionskonus 40 und eine im Stiel verankerte Expansionsschraube 39, die mit einem Drehmomentschlüssel 38 eingedreht wird, um die Expansionskräfte zu begrenzen. Der Prothesenkopf 4 besteht aus einer Gusslegierung (ISO 5832-4) und ist in verschiedenen Durchmessern 46 und Höhen 47 ausgeführt (Grössen a bis i Figur 4). Die Unterseite 23 des Kopfes bildet eine ebene Auflagefläche. Jeder Prothesenkopf besitzt die gleiche kugelförmige Lagerschale 11, die im Längsschnitt mehr als 180° gekrümmt ist und die einen gleichen Abstand zur Unterseite 23 vom Kopf aufweist. Zwischen dem Zentrum 12 der Lagerschale 11 und der Mittelachse 13 besteht ein Versatz 45 mit dem der Prothesenkopf 4 um das Zentrum 12 der Lagerschale 11 gedreht werden kann.

Damit der Prothesenkopf 4 wie in Figur 3 auf einer Resektionsfläche 37 aufliegt, diese möglichst genau abdeckt und eine für seine Funktion passende Höhe 47 aufweist, sind in ihren äusseren Abmessungen baugleiche Probierprothesen 6 vorgesehen, deren Stiele 7 und Köpfe 8 sich wie bei den eigentlichen Schaftprothesen 2 kombinieren lassen. Es besteht ebenfalls eine Kupplung 9 als festsetzbares Kugelgelenk 10 mit gleicher Lage vom Zentrum 12 und mit gleichem Versatz 45 zur Mittelachse im Prothesenkopf 8. Im Unterschied zur Schaftprothese 2 ist das Lager der Probierprothese 6 mit fünf Madenschrauben, die durch Bohrungen 43 in der Artikulationsfläche im eingesetzten Zustand mit einem Werkzeug 44 erreichbar sind, während dem Einsetzen in eine optimale Stellung drehbar gehalten und nach dem Erreichen dieser Stellung lösbar festsetzbar. In Figur 6 ist eine eingesetzte Probierprothese 6 gezeigt. Am Prothesenkopf 8 sind radial verlaufende Gewindebohrungen 43 angebracht, in denen Madenschrauben eingedreht sind, die das Lager im Kopf festsetzen. Fünf Madenschrauben sind über den Umfang verteilt, damit wenigstens zwei davon im eingesetzten Zustand mit dem Werkzeug 44 erreichbar sind. Eine weitere Bohrung 42 in der Artikulationsfläche ermöglicht den Zugang zu einem Gewinde für ein Ausziehwerkzeug 49 (Figur 7), an welches ein Gleithammer 50, 50a ansetzbar ist. Bei genügend grosser Lagerschale 11 der Probierprothese, lässt sich das Ausziehwerkzeug 49 in ein Gewinde am Kugelkopf vom Stiel 7 einschrauben, um die Ausziehkräfte direkt auf den Stiel 7 zu bringen und die Stellung des festgesetzten Gelenks 9 nicht zu gefährden.

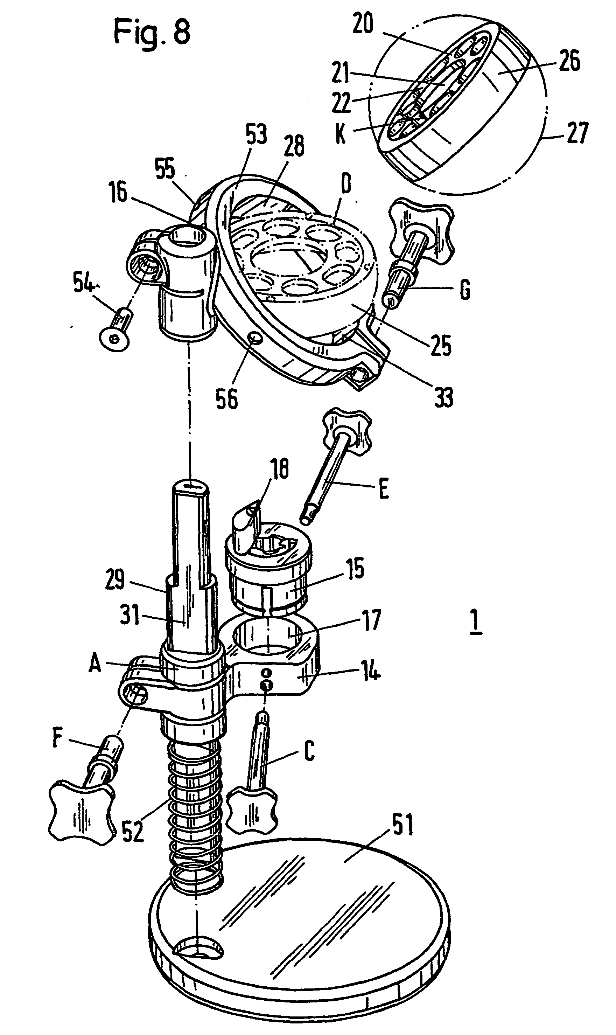

Die Montagevorrichtung 1 in Figur 8 besteht aus einem Ständer 29 mit Fuss 51. Erste Aufnahmevorrichtungen 14, 15 für Prothesenstiele sind mit einem Schlitten A verbunden, der an einer Führung 31 längs des Ständers 29 beweglich ist und dessen Gewicht mit einer Feder 52 teilweise kompensiert wird. Der Schlitten A kann in verschiedenen Höhenlagen mit einer Schraube F blockiert werden. Hülsen 15, die jeweils eine Aufnahmefläche und eine Nut 18 für eine bestimmte Stielgrösse und die dazugehörige Finne 19 besitzen, werden mit einer Schraube C in einer eindeutigen Lage auf dem Schlitten A, 14 befestigt. An der Hülse 15 greift eine Schraube E ein, mit der ein eingesetzter Stiel 3, 7 in wiederholbarer eindeutiger Lage klemmbar ist.

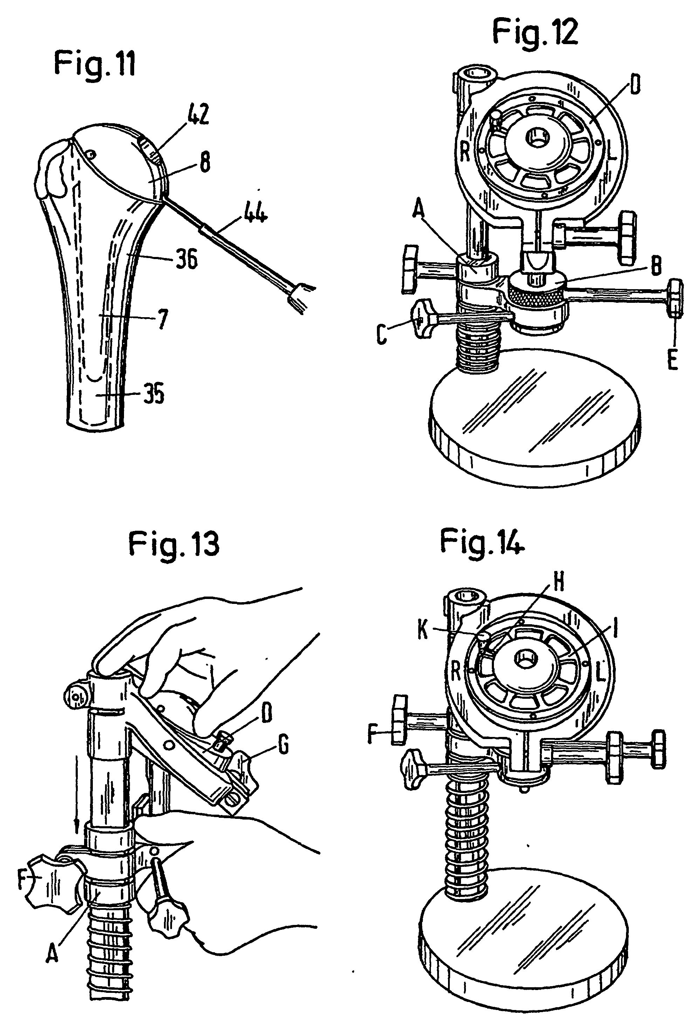

Eine zweite Aufnahmevorrichtung 16, die über eine Schraube 54 am Ende des Ständers 29 befestigt ist, trägt einen aufgeschnittenen Ring 53, dessen Durchmesser über eine Stellschraube G geringfügig veränderbar ist. In diesen Ring 53 ist eine schwimmende Scheibe D (punktiert gezeichnet) eingesetzt, die in ausgezogenen Linien 20 noch einmal ausserhalb dargestellt ist. Die schwimmende Scheibe D, 20 kann geführt im Ring 53 in eine beliebige Winkelstellung gedreht werden. Die äussere Lagerfläche 26 der schwimmenden Scheibe 20 entspricht dem Äquatorband einer Kugelfläche 27, für die eine ringförmige Gegenfläche 28 im aufgeschnittenen Ring 53 eingearbeitet ist. Durch Verstellen der Schraube G kann die schwimmende Scheibe D, 20 in einer beliebigen Winkelstellung blockiert werden. Die schwimmende Scheibe 20 hat eine mittlere Öffnung 21, durch welche Prothesenstiele 3, 7 ohne zu Berühren hindurchsteckbar sind. Ausserdem ist auf der schwimmenden Scheibe eine auf dem Umfang verschiebbare Drehscheibe I mit Marke H (Figur 14) angebracht, die mit einer Schraube K in einer vorgesehenen Winkellage blockiert werden kann. Am Ring 53 sind radial einsteckbare Stifte 55, 56 angebracht, die die schwimmende Scheibe D als Anschläge in einer vorgesehenen Standard Stellung, die einer Durchschnittsstellung von vielen Patienten entspricht, positionieren können.

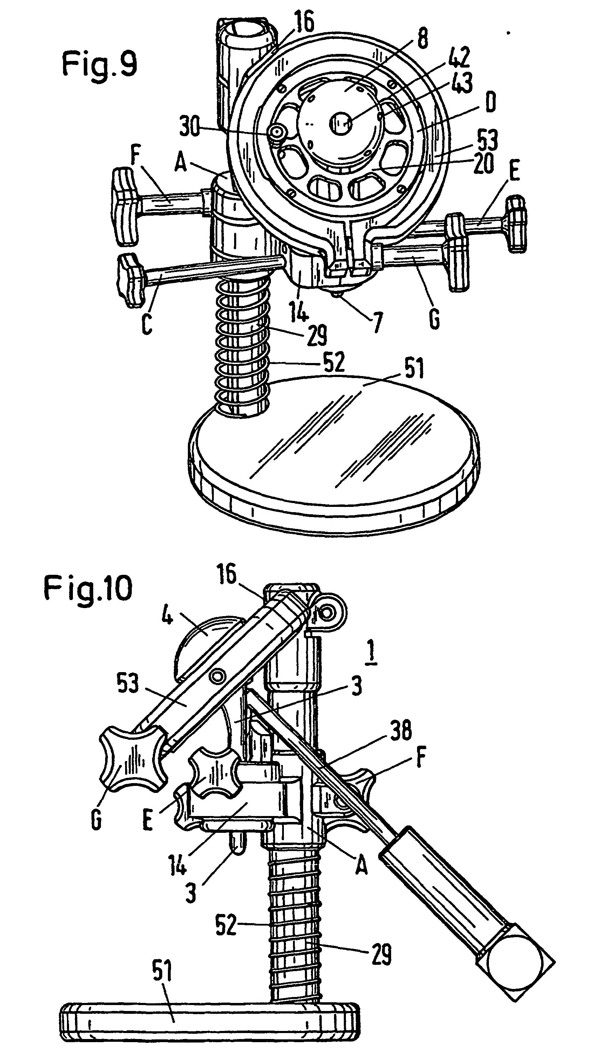

In Figur 9 ist eine Probierprothese mit ihrem Stiel 7 in der ersten Aufnahmevorrichtung 14 eingespannt und bestimmt mit ihrem Kopf 8 auf dessen Unterseite, die an einer Resektionsfläche abgegriffene Winkelstellung, welche die schwimmende Scheibe D, 20 bei vorsichtigem Absenken vom Schlitten A einnimmt. In dieser Stellung wird mit der Schraube 30, K eine Marke H an eine Markierung am Kopf 8 gedreht, die der Richtung vom Versatz 45 entspricht, und blockiert, um für die später in der Montagevorrichtung eingesetzte Schaftprothese 2 die Lage vom Versatz zu speichern.

In Figur 10 ist die Probierprothese inzwischen nach dem Lösen der Schraube E entfernt worden. Der Schlitten A wird zunächst in eine etwas höhere Position gebracht, um die baugleiche Schaftprothese 2 mit ihrem Stiel 3 in der ersten Aufnahmevorrichtung 14 zu positionieren und zu klemmen. Anschliessend wird der Schlitten G vorsichtig abgesenkt, damit der auf dem Stiel 3 schwenkbare Prothesenkopf 4 mit seiner Unterseite die Lage der blockierten schwimmenden Scheibe d.h. die Ebene der Resektionsfläche einnimmt. In dieser Neigung wird der Kopf 4 soweit gedreht bis seine Markierung für den Versatz mit der Marke H d.h. mit dem Versatz der Probierprothese übereinstimmt. In dieser Stellung wird die Expansionsschraube 39 mit einem Drehmomentschlüssel 38 angezogen, um das Lager 5, 11 kontrolliert festzusetzen. Die Schaftprothese 2 entspricht jetzt der vorher an Ort kontrollierten Probierprothese 6 und kann implantiert werden.

In den Figuren 11 bis 14 sind die Schritte an der Probierprothese in einer Folge aufgezeigt:
- In Figur 14 wird der Kopf 8 einer passenden Probierprothese, die mit ihrem Stiel 7 in einer eindeutigen Position im Markkanal 35 eines Humerus eingesetzt ist, nach der Lage der Resektionsfläche ausgerichtet und relativ zum Stiel 7 in dieser Position mit dem Werkzeug 44 durch Eindrehen von Madenschrauben gesichert. Mit einem Ausziehwerkzeug 49 wird der Schaft durch eine Bohrung 42 gefasst und ausgezogen. Die restlichen Madenschrauben werden ebenfalls angezogen zur besseren Sicherung der Winkelstellung des Kopfes 8.
- In Figur 12 ist die Probierprothese lose auf der schwimmenden Scheibe D aufgesetzt worden. Der Schlitten A wird zunächst nach unten gefahren, um eine zum Stiel 7 passende Hülse B, 15 in der ersten Aufnahmevorrichtung einzulegen und mit Schraube C zu sichern. Anschliessend wird der Schlitten A nach oben gefahren und der Stiel 7 in der Hülse B, 15 positioniert und mit Schraube E gesichert.
- In Figur 13 wird der Schlitten A vorsichtig nach unten gefahren, damit sich die schwimmende Scheibe D nach der Neigung der Unterseite des Kopfes ausrichtet und die Neigung durch Anziehen der Schraube G fixiert.
- In Figur 14 wird in der geneigten Ebene der schwimmenden Scheibe D eine Drehscheibe mit einer Marke H an den Ort verschoben, der auf dem Kopf 8 die Lage vom Versatz anzeigt, und die Drehscheibe I anschliessend mit einer Schraube K gegenüber der schwimmenden Scheibe D gesichert. Der Zustand entspricht jetzt der Darstellung von Figur 9. Die Stellung der Probierprothese ist in der Montagevorrichtung 1 gespeichert und die Probierprothese kann nach dem Lösen der Klemmschraube E entfernt werden.

## Patentansprüche

1. Baukasten mit einer Montagevorrichtung (1) zum Montieren von Schaftprothesen, mit Schaftprothesen (2), die aus verschieden grossen Stielen (3, S₁, S_{2'} S₃, S₄) und aus verschieden grossen Prothesenköpfen (4, a, b, c, d, e, f, g, h, i) zusammensetzbar sind, wobei zwischen Stiel (3) und Prothesenkopf (4) eine festsetzbare Kupplung (5) besteht, die unterschiedliche Positionen und Winkellagen zwischen Prothesenkopf (4) und Stiel (3) zulässt, mit Probierprothesen (6, 7, 8), die analog zu den Schaftprothesen in unterschiedlichen Grössen zusammensetzbar sind und eine lösbar festsetzbare Kupplung (9) aufweisen, welche bei der in einem Knochen eingesetzten Probierprothese (6) festsetzbar ist, um eine optimale Position und Winkellage des Kopfes (8) der Probierprothese zu ihrem Stiel (7) festzuhalten, welche nach dem Entfernen der Probierprothese (6) mit dieser auf die Montagevorrichtung (1) übertragbar ist, die erste Aufnahmevorrichtungen (14) aufweist, mit denen jede Stielgrösse (S₁, S₂, S₃, S₄) in einer eindeutigen und wiederholbaren Lage positionierbar und klemmbar ist, und die mindestens eine zweite Aufnahmevorrichtung (16) aufweist, die geführt relativ zu den ersten Aufnahmevorrichtungen (14) verschiebbar und in eine eindeutige Winkellage und Position des Kopfes (8) einer in der ersten Aufnahmevorrichtung (14) geklemmten Probierprothese (6, 7, 8) nachführbar und dort fixierbar ist, um nach dem Entfernen der Probierprothese (6) einen gleich grossen Stiel (3) und einen gleich grossen Prothesenkopf (4) in der gleichen Position und Winkellage zueinander auszurichten und zu verbinden.

2. Baukasten nach Anspruch 1, **dadurch gekennzeichnet, dass** die Schaftprothesen (2) Schulterprothesen sind.

3. Baukasten nach Anspruch 2, **dadurch gekennzeichnet, dass** die Schulterprothesen eine Kupplung (5) in Form eines festsetzbaren Kugelgelenks (10) zwischen Stiel (3) und Prothesenkopf (4) aufweisen, wobei die Lagerschale (11) des Kugelgelenks (10) im Prothesenkopf (4) angeordnet ist.

4. Baukasten nach Anspruch 2, **dadurch gekennzeichnet, dass** die Probierprothesen eine Kupplung in Form eines festsetzbaren Kugelgelenks (10) zwischen Stiel (7) und Prothesenkopf (8) in der gleichen Lage wie die Schaftprothesen aufweisen, wobei die Lagerschale (11) im Prothesenkopf (8) angeordnet ist und der vom Stiel (7) vorstehende Kugelkörper durch im Prothesenkopf (8) radial zustellbare Madenschrauben zunächst gefangen und anschliessend lösbar festsetzbar ist.

5. Baukasten nach Anspruch 3 oder 4, **dadurch gekennzeichnet, dass** das Zentrum (12) der Lagerschale (11) radial zur Mittelachse (13) des Prothesenkopfes (4, 8) versetzt ist.

6. Montagevorrichtung für einen Baukasten nach einem der Ansprüche 1 bis 5, die erste Aufnahmevorrichtungen (14) aufweist, mit denen jede Stielgrösse (S₁, S₂, S₃, S₄) von verschieden großen Schaftprothesen in einer eindeutigen und wiederholbaren Lage positionierbar und klemmbar ist, und die mindestens eine zweite Aufnahmevorrichtung (16) aufweist, die geführt relativ zu den ersten Aufnahmevorrichtungen (14) verschiebbar und in eine eindeutige Winkellage und Position des Kopfes (8) einer in der ersten Aufnahmevorrichtung (14) geklemmten Probierprothese (6, 7, 8) nachführbar und dort fixierbar ist, um nach dem Entfernen der Probierprothese (6) einen gleich grossen Stiel (3) und einen gleich grossen Prothesenkopf (4) in der gleichen Position und Winkellage zueinander auszurichten und zu verbinden.

7. Montagevorrichtung nach Anspruch 6, **dadurch gekennzeichnet, dass** die ersten Aufnahmevorrichtungen (14) aus einer Aufnahme (17) für Hülsen (15) mit einer gleichen Aussenform und mit jeweils einer eindeutigen Positioniermöglichkeit (18, 19) auf ihrer Innenseite für eine bestimmte Stielgrösse (S₁, S₂, S₃, S₄) bestehen.

8. Montagevorrichtung nach Anspruch 6 oder 7, **dadurch gekennzeichnet, dass** die zweite Aufnahmevorrichtung (16) mit einer schwimmenden Scheibe (20) versehen ist, die eine Bohrung (21) zum Hindurchführen eines Stiels (3, 7) und eine Auflageebene (22) für die Unterseite (23, 24) der Prothesenköpfe (4, 8) aufweist, dass die schwimmende Scheibe (20) eine äussere Führungsfläche (25) aufweist, die dem Äquatorband (26) einer Kugelfläche (27) entspricht, und dass diese äussere Führungsfläche (25) durch eine ringförmige Gegenfläche (28) derart gefangen ist, dass sie nur Drehungen in der Kugelfläche (27) durchführen kann, nach denen sie fixierbar ist.

9. Montagevorrichtung nach Anspruch 7, **dadurch gekennzeichnet, dass** Mittel (30) vorhanden sind, um die Winkellage der Mittelachse (13) eines Prothesenkopfes (3, 4), dessen Zentrum (12) der Lagerschale (11) zur Mittelachse (13) radial versetzt ist, auf der schwimmenden Scheibe (20) zu markieren.

10. Montagevorrichtung nach einem der Ansprüche 6 bis 9, **dadurch gekennzeichnet, dass** sie als Ständer (29) ausgeführt ist, der eine Führung (31) für die ersten Aufnahmevorrichtungen (14) und für die zweite Aufnahmevorrichtung (16) bildet, wobei die in den ersten Aufnahmevorrichtungen (14) einsetzbaren Stiele (7, 3) mit ihrer Achse (32) annähernd parallel zum Ständer verlaufen.

11. Montagevorrichtung nach einem der Ansprüche 8 bis 10, **dadurch gekennzeichnet, dass** die räumliche Ausrichtung der ringförmigen Gegenfläche (28) so zur Achse (32) eines eingesetzten Stiels (7, 3) vorgenommen ist, dass die Ebene (33) des grössten Durchmessers eine Winkellage zur Stielachse (32) aufweist, die einer durchschnittlichen Winkellage eines zu einem Gelenkkopf (8, 4) gleichwertigen natürlichen Gelenkstückes zu der Achse (34) seines zugehörigen Markkanals (35) entspricht.

## Claims

1. Modular system with an assembly apparatus (1) for the assembly of shaft prostheses, having shaft prostheses (2) which can be assembled from stems (3, S₁, S₂, S₃, S₄) of different sizes and prosthesis heads (4, a, b, c, d, e, f, g, h, i) of different sizes, with a fixable coupling (5) existing between the stem (3) and the prosthesis head (4) which allows different positions and angular orientations between the prosthesis head (4) and the stem (3); and also having test prostheses (6, 7, 8) capable of being assembled in different sizes analogously to the shaft prostheses and having a releasably fixable coupling (9) which can be fixed in the test prosthesis (6) which is inserted in a bone in order to hold firmly an ideal position and angular orientation of the head (8) of the test prosthesis with respect to its stem (7) which, after the removal of the test prosthesis (6), can be transferred with the latter to the assembly apparatus (1), the assembly apparatus having first receivers (14) by means of which each stem size (S₁, S₂, S₃, S₄) can be positioned and clamped in a unique and reproducible position, and at least one second receiver (16) which is guidedly displaceable relative to the first receivers (14) and guidable into a unique angular orientation and position of the head (8) of a test prosthesis (6, 7, 8) which is clamped in the first receiver (14) and can be fixed there in order to mutually orient and to connect an equally large stem (3) and an equally large prosthesis head (4) in the same position and angular orientation after the removal of the test prosthesis (6).

2. Modular system in accordance with claim 1, **characterised in that** the shaft prostheses (2) are shoulder prostheses.

3. Modular system in accordance with claim 2, **characterised in that** the shoulder prostheses have a coupling (5) in the form of a fixable ball joint (10) between the stem (3) and the prosthesis head (4), with the bearing shell (11) of the ball joint (10) being arranged in the prosthesis head (4).

4. Modular system in accordance with claim 2, **characterised in that** the test prostheses have a coupling in the form of a fixable ball joint (10) between the stem (7) and the prosthesis head (8) in the same position as the shaft prostheses, with the bearing shell (11) being arranged in the prosthesis head (8) and with it being possible first to capture and then to releasably fix the spherical body projecting from the stem (7) by grub screws which can be fed in radially in the prosthesis head (8).

5. Modular system in accordance with claim 3 or claim 4, **characterised in that** the centre (12) of the bearing shell (11) is offset radially with respect to the central axis (13) of the prosthesis head (4, 8).

6. Assembly apparatus for a modular system in accordance with any one of claims 1 to 5 which has the first receivers (14) by means of which each stem size (S₁, S₂, S₃, S₄) of shaft prostheses of different sizes can be positioned and clamped in a unique and reproducible position, and which has at least one second receiver (16) which is guidedly displaceable relative to the first receivers (14) into a unique angular orientation and position of the head (8) of a test prosthesis (6, 7, 8) which is clamped in the first receiver (14) and can be fixed there in order to mutually orient and to connect an equally large stem (3) and an equally large prosthesis head (4) in the same position and angular orientation after the removal of the test prosthesis (6).

7. Assembly apparatus in accordance with claim 6, **characterised in that** the first receivers (14) consist of a seat (17) for sleeves (15) with a similar outer shape and with in each case a unique positioning possibility (18, 19) on its inner side for a definite stem size (S₁, S₂, S₃, S₄).

8. Assembly apparatus in accordance with claim 6 or claim 7, **characterised in that** the second receiver (16) is provided with a floating disc (20) which has a bore (21) for the passing through of a stem (3, 7) and a contact plane (22) for the lower side (23, 24) of the prosthesis heads (4, 8); **in that** the floating disc (20) has an outer guide surface (25) which corresponds to the equatorial band (26) of a spherical surface (27); and **in that** this outer surface (25) is captured by a ring shaped counter-surface (28) in such a manner that it can carry out rotations only in the spherical surface (27), after which it can be fixed.

9. Assembly apparatus in accordance with claim 7, **characterised in that** means (30) are present in order to mark on the floating disc (20) the angular orientation of the central axis (13) of a prosthesis head (3, 4), the centre (12) of the bearing shell (11) of which is radially offset with respect to the central axis (13).

10. Assembly apparatus in accordance with any one of claims 6 to 9, **characterised in that** it is designed as a stand (29) which forms a guide (31) for the first receivers (14) and for the second receiver (16), with the stems (7, 3), which are insertable into the first receivers (14), extending with their axis (32) approximately parallel to the stand.

11. Assembly apparatus in accordance with any one of the claims 8 to 10, **characterised in that** the spatial orientation of the ring shaped counter-surface (28) is carried out relative to the axis (32) of an inserted stem (7, 3) such that the plane (33) of the largest diameter has an angular orientation relative to the stem axis (32) which corresponds to an average angular orientation of a natural joint piece equivalent to a joint head (8, 4) relative to the axis (34) of its associated medullary canal (35).

## Revendications

1. Système d'assemblage modulaire comportant un dispositif de montage (1) pour l'assemblage de prothèses à broches, les prothèses à broches (2) pouvant être constituées à partir de tiges (3, S₁, S₂, S₃, S₄) de différentes tailles et de têtes de prothèses (4, a, b, c, d, e, f, g, h, i) de différentes tailles, dans lequel il est prévu, entre une tige (3) et une tête de prothèse (4), un accouplement (5) qui peut être fixé et qui autorise différentes positions et différentes positions angulaires relatives entre la tête de prothèse (4) et la tige (3) ; des prothèses d'essai (6, 7, 8), qui peuvent être constituées, de manière analogue aux prothèses à broches, en différentes tailles et qui présentent un accouplement (9) qui peut être fixé et qui est démontable, qui peut être fixé lorsque la prothèse d'essai (6) a été introduite dans un os, afin d'assurer une position et une position angulaire relative optimales de la tête (8) de la prothèse d'essai par rapport à sa tige (7), ces positions pouvant être transmises, après avoir éloigné la prothèse d'essai (6), par celle-ci au dispositif de montage (1), qui présente des premiers dispositifs récepteurs (14) à l'aide desquels chaque taille de tige (S₁, S₂, S₃, S₄) peut être positionnée et bloquée dans une position définie et reproductible, et qui présente au moins un deuxième dispositif récepteur (16) qui peut être déplacé d'une manière guidée relativement aux premiers dispositifs récepteurs (14) et qui peut être orienté vers une position angulaire relative et une position définie de la tête (8) d'une prothèse d'essai (6, 7, 8) bloquée dans le premier dispositif récepteur (14) et y être immobilisé, afin de pouvoir, après l'éloignement de la prothèse d'essai (6) aligner et relier entre elles une tige (3) de la même taille et une tête de prothèse (4) de la même taille dans la même position et dans la même position angulaire relative.

2. Système d'assemblage modulaire selon la revendication 1, **caractérisé en ce que** les prothèses à broches (2) sont des prothèses pour épaules.

3. Système d'assemblage modulaire selon la revendication 2, **caractérisé en ce que** les prothèses pour épaules présentent un accouplement (5) sous la forme d'une articulation sphérique (10) qui peut être fixée entre la tige (3) et la tête de prothèse (4), et la coque d'appui (11) de l'articulation sphérique (10) étant placée dans la tête de prothèse (4).

4. Système d'assemblage modulaire selon la revendication 2, **caractérisé en ce que** les prothèses d'essai présentent un accouplement sous la forme d'une articulation sphérique (10) qui peut être fixée entre la tige (7) et la tête de prothèse (8) dans la même position que les prothèses à broches, la coque d'appui (11) étant placée dans la tête de prothèse (8) et le corps sphérique faisant saillie à partir de la tige (7) pouvant être d'abord retenu par des vis sans tête, qui peuvent être radialement avancées dans la tête de prothèse (8), et pouvant être ensuite fixé de manière détachable.

5. Système d'assemblage modulaire selon la revendication 3 ou 4, **caractérisé en ce que** le centre (12) de la coque d'appui (11) est décalé radialement par rapport à l'axe médian (13) de la tête de prothèse (4, 8).

6. Dispositif de montage pour un système d'assemblage selon l'une des revendications 1 à 5, qui présente des premiers dispositifs récepteurs (14), au moyen desquels chaque taille de tige (S₁, S₂, S₃, S₄) de prothèses à broches de tailles différentes peut être positionnée et bloquée dans une position définie et reproductible, et qui présente au moins un deuxième dispositif récepteur (16) qui peut être déplacé d'une manière guidée relativement aux premiers dispositifs récepteurs (14) et qui peut être orienté vers une position angulaire relative et une position définies de la tête (8) d'une prothèse d'essai (6, 7, 8) bloquée dans le premier dispositif récepteur (14) et y être immobilisé, afin de pouvoir, après l'éloignement de la prothèse d'essai (6), aligner et relier entre elles une tige (3) de la même taille et une tête de prothèse (4) de la même taille, dans la même position et dans la même position angulaire relative.

7. Dispositif de montage selon la revendication 6, **caractérisé en ce que** les premiers dispositifs récepteurs (14) consistent en un logement (17) pour des douilles (15) ayant une forme extérieure identique, et avec chaque fois une possibilité définie de positionnement (18, 19) sur leur côté intérieur pour une taille déterminée de tige (S₁, S₂, S₃, S₄).

8. Dispositif de montage selon la revendication 6 ou 7, **caractérisé en ce que** le deuxième dispositif récepteur (16) est pourvu d'un disque flottant (20), qui présente un alésage (21) pour le passage d'une tige (3, 7) et un plan de support (22) pour le côté inférieur (23, 24) des têtes de prothèse (4, 8), **en ce que** le disque flottant (20) présente une surface extérieure de guidage (25), qui correspond à la bande équatoriale (26) d'une surface sphérique (27), et **en ce que** cette surface extérieure de guidage (25) est retenue par une contre-surface annulaire (28) de telle sorte qu'elle ne puisse exécuter que des rotations dans la surface sphérique (27), après lesquelles elle peut être immobilisée.

9. Dispositif de montage selon la revendication 7, **caractérisé en ce que** des moyens (30) sont prévus pour pouvoir repérer, sur le disque flottant (20), la position angulaire relative de l'axe médian (13) d'une tête de prothèse (3, 4), dont le centre (12) de la coque d'appui (11) est décalé radialement par rapport à l'axe médian (13).

10. Dispositif de montage selon l'une des revendications 6 à 9, **caractérisé en ce qu'**il est réalisé en tant que colonne (29), qui constitue un élément de guidage (31) pour les premiers dispositifs récepteurs (14) et pour le deuxième dispositif récepteur (16), et les tiges (7, 3), qui peuvent être introduites dans les premiers dispositifs récepteurs (14), s'étendant par leur axe (32) sensiblement parallèlement à la colonne.

11. Dispositif de montage selon l'une des revendications 8 à 10, **caractérisé en ce que** l'orientation spatiale de la contre-surface annulaire (28) est effectuée par rapport à l'axe (32) d'une tige (7, 3) insérée de telle manière que le plan (33) du diamètre le plus grand présente une position angulaire relative par rapport à l'axe de tige (32), qui corresponde à une position angulaire relative moyenne d'une partie d'articulation naturelle équivalente à une tête d'articulation (8, 4), par rapport à l'axe (34) de son canal médullaire (35).
